# EUROPÄISCHE PATENTSCHRIFT

(11) **EP 3 773 364 B1**
(45) Veröffentlichungstag und Bekanntmachung des Hinweises auf die Patenterteilung: **11.05.2022**
(21) Anmeldenummer: 19714151.8
(22) Anmeldetag: 22.03.2019
(51) Int. Cl.: A61F 5/01

(54) **STABILISIERUNGSSTAB FÜR EIN ORTHOPÄDISCHES HILFSMITTEL**
STABILISING ROD FOR AN ORTHOPAEDIC AID
BARRE STABITLISATRICE DESTINÉE À UN AUXILIAIRE ORTHOPÉDIQUE

(30) Priorität: 27.03.2018 DE 102018204640
(43) Veröffentlichungstag der Anmeldung: 17.02.2021
(73) Patentinhaber: Bauerfeind AG, 07937 Zeulenroda-Triebes (DE)
(72) Erfinder: BAUERFEIND, Hans B., 07937 Zeulenroda-Triebes (DE)
(74) Vertreter: Wohlfahrt, Jan Günther
(86) Internationale Anmeldenummer: PCT/EP2019/057289
(87) Internationale Veröffentlichungsnummer: WO 2019/185485

(56) Entgegenhaltungen:
- WO-A1-2018/232029
- WO-A2-2014/001748
- US-A- 1 142 045
- US-A- 3 531 807
- US-A1- 2005 038 367
- US-A1- 2007 167 891

## Beschreibung

Die vorliegende Erfindung betrifft Stabilisierungsstäbe für ein orthopädisches Hilfsmittel, insbesondere Kniebandagen, und orthopädische Hilfsmittel, umfassend mindestens einen erfindungsgemäßen Stabilisierungsstab.

Der Einsatz von Stabilisierungsstäben in orthopädischen Hilfsmitteln ist bekannt. Kniegelenksbandagen, kurz Kniebandagen, mit seitlichen Stabilisierungsstäben sind aus der WO 2011/035885 A1 und der DE 3637 879 A1 bekannt. Dabei werden Stabilisierungsstäbe aus Metall verwendet, die durch den Aufbau als Federbandstäbe, also flachgepressten Windungen einer Schraubenfeder, biegsam sind. Da solche Stabilisierungsstäbe jedoch häufig an das Textil des orthopädischen Hilfsmittels angeschweißt werden, müssen zumindest Teilbereiche der metallischen Federbandstäbe mit einem anschweißbaren Kunststoff kaschiert werden. Darüber hinaus kann das Metall das anliegende Textil zerstören. Die eingesetzten flachen Spiralfedern sind dazu über den ganzen Verlauf der Feder gleich elastisch und der Grad der Einbeugung kann nicht limitiert werden.

US 1,142,045 offenbart einen Stab bestehend aus einem widerstandsfähigen Streifen mit queren Schlitzen, welche, von den Kanten aus gesehen, nach innen gerichtet sind, an seinem Endabschnitt, wobei die Schlitze eine graduell ansteigende Länge in Richtung des Endes des Stabes aufweisen, wobei dadurch die Widerstandsfähigkeit des Endabschnittes des Stabes graduell erhöht wird.

Das der vorliegenden Erfindung zugrunde liegende technische Problem ist die Bereitstellung von verbesserten Stabilisierungsstäben für ein orthopädisches Hilfsmittel, insbesondere Kniebandagen, die mindestens so gut funktionieren wie die Stabilisierungsstäbe aus dem Stand der Technik, aber dabei einfacher und billiger zu produzieren sind, gut verschweißbar sind, leichter sind und oder das anliegende Textil schonen.

Die vorliegende Erfindung löst dieses technische Problem durch einen Stabilisierungsstab nach Anspruch 1.

Insbesondere löst die vorliegende Erfindung das ihr zugrunde liegende technische Problem durch einen Stabilisierungsstab für ein orthopädisches Hilfsmittel, wobei der Stabilisierungsstab einen biegsamen Abschnitt aufweist, wobei der biegsame Abschnitt mindestens eine Materialaussparung aufweist.

Bevorzugt weist der biegsame Abschnitt mehrere Materialaussparungen auf. Der biegsame Abschnitt kann sich über die gesamte Länge des Stabilisierungsstabs erstecken oder nur eine Teillänge des Stabilisierungsstabs ausmachen.

Die vorliegende Erfindung löst das ihr zugrunde liegende technische Problem durch einen Stabilisierungsstab für ein orthopädisches Hilfsmittel, insbesondere für eine Kniebandage, wobei der Stabilisierungsstab in Längsrichtung einen ersten Abschnitt und einen zweiten Abschnitt aufweist, wobei der erste Abschnitt und der zweite Abschnitt über einen dritten, biegsamen Abschnitt miteinander verbunden sind, wobei zumindest der dritte, biegsame Abschnitt mehrere Materialaussparungen aufweist, wobei die Materialaussparungen umso tiefer sind, je weiter sie vom Mittelbereich des dritten biegsamen Abschnitts entfernt sind, wobei die Materialaussparungen durch zwischen den Materialaussparungen liegenden zahnartigen Materialerhebungen gebildet werden.

Insbesondere löst die vorliegende Erfindung das ihr zugrunde liegende technische Problem durch einen Stabilisierungsstab für ein orthopädisches Hilfsmittel, insbesondere für eine Kniebandage, wobei der Stabilisierungsstab in Längsrichtung einen ersten Abschnitt und einen zweiten Abschnitt aufweist, wobei der erste Abschnitt und der zweite Abschnitt über einen dritten, biegsamen Abschnitt miteinander verbunden sind, dadurch gekennzeichnet, dass der dritte, biegsame Abschnitt mehrere Materialaussparungen aufweist.

Bevorzugt weist der dritte biegsame Abschnitt mehrere Materialaussparungen auf.

Bevorzugt weist der biegsame Abschnitt eine Vielzahl von Materialaussparungen auf. Bevorzugt befindet sich die Vielzahl von Aussparungen auf einer Seite des Stabilisierungsstabs. Bevorzugt ist dabei die Seite des Stabilisierungsstabs, die bei einem Verbiegen des Stabilisierungsstabes die innere, konkave Seite bildet mit der Vielzahl von Aussparungen versehen.

Bevorzugt weisen der erste Abschnitt und der zweite Abschnitt nicht mehrere Materialaussparungen auf. Der erste Abschnitt und/oder der zweite Abschnitt können alternativ jedoch auch Materialaussparungen aufweisen, zum Beispiel wenn auch diese Abschnitte biegsam ausgestaltet werden sollen.

Durch die Länge und Positionierung des biegsamen Abschnitts kann in vorteilhafter Weise Lage und Stärke der Biegsamkeit des Stabilisierungsstabes bestimmt werden.

Bevorzugt weist der dritte biegsame Abschnitt drei bis fünfzehn Materialaussparungen auf. Bevorzugt weist der dritte biegsame Abschnitt vier bis zehn Materialaussparungen auf. Bevorzugt weist der dritte biegsame Abschnitt vier bis fünfzehn Materialaussparungen auf. Bevorzugt weist der dritte biegsame Abschnitt mindestens vier Materialaussparungen auf. Bevorzugt weist der dritte biegsame Abschnitt sieben bis zwölf Materialaussparungen auf.

Insbesondere können die Materialaussparungen als Einschnitte ausgebildet sein. Der dritte Abschnitt ist also bevorzugt durch die mehreren Einschnitte zahnschienförmig ausgebildet.

Es zeigte sich überraschender Weise, dass durch die erfindungsgemäße Materialaussparungen mit den dazwischen liegenden Zähnen der Beugewinkel des Stabilisierungsstabes limitiert werden kann, wenn durch das Biegen es Stabes die Zähne aneinanderstoßen. Wenn der Stabilisierungsstab soweit gebogen wird, dass alle zwischen den Einschnitten liegenden Zähne aneinanderstoßen, ist eine weitere Beugung nur noch durch ein Verdrehen des Stabilisierungsstabs möglich, was die das orthopädisches Hilfsmittel tragende Person direkt spürt und somit ein Feedback zur Beugungsbegrenzung erhält.

Es zeigte sich überraschender Weise weiter, dass durch solche Materialaussparungen eine gute und ausreichende Biegsamkeit des Stabilisierungsstabs erreicht werden kann, ohne dass der Stabilisierungsstab als Federbandstab ausgebildet sein muss. Weiterhin zeigte sich, dass es dabei für die Biegsamkeit ausreichend ist, dass nur ein Mittelbereich des Stabilisierungsstabs Materialaussparungen aufweist. Da kein Federbandstab, insbesondere durchgehender Federbandstab notwendig ist, kann in vorteilhafter Weise auf Metall und/oder das kreisförmig überlappende Federband verzichtet werden, wodurch der Stabilisierungsstab das anliegende Textil schont. Auch kann der Stabilisierungsstab in vorteilhafter Weise einstückig und aus Kunststoff hergestellt werden, wodurch ein aufwendiger Kaschierungsschritt entfällt und der Stabilisierungsstab leichter ausgestaltet werden kann.

Durch die Position des biegsamen Abschnitts entlang der Länge des Stabilisierungsstabs kann der biegsame Bereich des Stabilisierungsstabs bestimmt werden.

Wenn der biegsame Abschnitt sich über die gesamte Länge des Stabilisierungsstabs erstreckt, kann der biegsame oder besonders biegsame Beriech des Stabilisierungsstabs durch die Materialdicke der jeweiligen Abschnitte bestimmt werden, indem der besonders biegsame Abschnitt eine geringere Materialdicke aufweist.

In einer bevorzugten Ausführungsform ist der Stabilisierungsstab einstückig. In einer bevorzugten Ausführungsform besteht der Stabilisierungsstab aus einem einzigen Material. In einer bevorzugten Ausführungsform besteht der Stabilisierungsstab aus einem Kunststoff.

Alternativ kann aber auch beispielsweise vorgesehen sein, dass in den Kunststoff des Stabilisierungsstabs eine Sehne aus Metall eingearbeitet ist. Auch wenn die Sehne aus Metall ist, ist sie dennoch leichter als ein Federbandstab. Wenn in den Stabilisierungsstab eine Sehne aus einem härteren Material als das Material des Stabilisierungsstabs, insbesondere eine Sehne aus Metall, eingebettet ist, kann der Stabilisierungsstab dünner ausgestaltet werden und behält dennoch seine Rückstellkraft.

Bevorzugt sind die Materialaussparungen umso tiefer, je weiter sie vom Mittelbereich des biegsamen Abschnitts entfernt sind.

Bevorzugt sind die Materialaussparungen im dritten Abschnitt umso tiefer, je weiter sie vom Mittelbereich des dritten biegsamen Abschnitts entfernt sind.

Bevorzugt sind die Materialaussparungen nur auf einer Seite des dritten Abschnitts.

Bevorzugt ist der dritte Abschnitt gegenüber dem ersten und zweiten Abschnitt verjüngt.

Bevorzugt ist der dritte Abschnitt gegenüber dem ersten und zweiten Abschnitt in Bezug auf die Breite des Abschnitts, insbesondere die Breite des Grundkörpers verjüngt.

Bevorzugt sind die Materialaussparungen, insbesondere Einschnitte, nicht kreisrund ausgeformt, sondern sind zum Stabilisierungsstabrand hin schmaler und bilden insbesondere bevorzugt die Form eines Schlüssellochs. Dies wird besonders bevorzugt dadurch erreicht, dass die Materialaussparungen durch zwischen den Materialaussparungen liegenden zahnartigen Materialerhebungen, gebildet werden, wobei bevorzugt diese zahnartigen Materialerhebungen hammerartig oder reißverschlusszahnartig geformt sind, also insbesondere zur Kante des Stabilisierungsstabs hin länger sind als zur Mitte des Grundkörpers hin. Eine solche nicht kreisrund, sondern schlüssellochartig ausgeformte Materialaussparung, insbesondere Einschnitt, hat den Vorteil, dass Spannungsspitzen im Material beim Biegen des Stabilisierungsstabs verringert werden.

Bevorzugt sind der erste Abschnitt und der zweite Abschnitt weniger biegsam als der dritte Abschnitt. Wenn der erfindungsgemäße Stabilisierungsstab bevorzugt in einer Kniebandage verwendet wird, so ist er insbesondere so an der Kniebandage angebracht, dass der dritte Abschnitt auf Höhe des Kniegelenks positioniert ist. Genau in diesem Bereich muss der Stabilisierungsstab biegsam sein, so dass es ausreichend ist, wenn der erste Abschnitt und der zweite Abschnitt weniger biegsam sind.

Bevorzugt ist die Breite des dritten, biegsamen Abschnitts kleiner als die Breite des ersten Abschnitts und die Breite des zweiten Abschnitts des Stabilisierungsstabs.

Bevorzugt hat der Stabilisierungsstab eine Länge, eine Breite und ein Höhe, wobei die Breite des ersten Abschnitts und die Breite des zweiten Abschnitts größer ist als die Höhe des Stabilisierungsstabes. Bevorzugt hat der Stabilisierungsstab eine Länge, eine Breite und ein Höhe, wobei die Breite des ersten Abschnitts und die Breite des zweiten Abschnitts größer ist als die Höhe des Stabilisierungsstabes und wobei die Breite des dritten, biegsamen Abschnitts kleiner ist als die Breite des ersten Abschnitts und die Breite des zweiten Abschnitts.

Bevorzugt weist der Stabilisierungsstab mindestens eine längsverlaufende Nut, insbesondere ein oder zwei längsverlaufende Nuten auf. Bevorzugt weist der dritte mittlere Bereich des Stabilisierungsstabs, insbesondere im Grundkörper, mindestens eine längsverlaufende Nut, insbesondere ein oder zwei längsverlaufende Nuten auf. Solche Nuten ermöglichen es in vorteilhafter Weise den Stabilisierungsstab nicht nur entlang der Verzahnung zu biegen, sondern auch in eine um 90 ° versetzte Richtung zu biegen, was insbesondere beim An- und Ausziehen einer Bandage, die einen solchen Stabilisierungsstab aufweist, vorteilhaft ist.

In einer bevorzugten Ausführungsform weist der dritte, mittlere Abschnitt eine gleiche oder größere Materialstärke in Bezug auf die Höhe des Stabilisierungsstabs, bezogen auf die Materialstärke des ersten und des zweiten Abschnitts, auf. Alternativ kann auch vorgesehen sein, die Höhe des dritten mittleren Bereichs zu variieren, beispielsweise wellenförmig zu variieren. Bevorzugt kann vorgesehen sein, dass der mittlere Bereich an den Stellen, an denen Materialaussparungen vorgesehen sind, verdickt oder verdünnt ist, insbesondere verdickt ist und an den Stellen, an denen sich zahnartigen Materialerhebungen anschließen, verdünnt oder verdickt, insbesondere verdünnt ist, wodurch sich eine wellenförmige Form ergibt.

In einer bevorzugten Ausführungsform ist der Grundkörper des Stabilisierungsstabs im Übergangsbereich vom ersten Abschnitt zum dritten Abschnitt und/oder vom zweiten Abschnitt zum dritten Abschnitt in der Höhe verdickt. Dies prägt in vorteilhafter Weise die durch die Verzahnung bereits definierte Biegestelle besser aus. Die Verdickung kann bevorzugt als Wulst ausgeformt sein.

In einer bevorzugten Ausführungsform ist der Grundkörper des Stabes zumindest bereichsweise keilförmig ausgebildet. Besonders bevorzugt ist der Grundkörper im dritten mittleren Bereich keilförmig ausgebildet, insbesondere derartig ausgebildet, dass er sich in Richtung der zahnartigen Erhebungen verjüngt. Diese Ausgestaltung fördert in vorteilhafter Weise das leichtere Einbiegen des Stabes, da die Stauchung des Materials im Bereich der Biegestelle am Größten ist.

In einer bevorzugten Ausführungsform hat der Grundkörper im dritten, mittleren Bereich eine größere Materialdicke, ist also höher als der erste und/oder der zweite Bereich.

In einer bevorzugten Ausführungsform sind zumindest Teilbereiche der Oberfläche des Stabilisierungsstabs nicht glatt sondern angeraut. Dies hat den Vorteil, dass ein Quietschen des Stabes, wenn er sich beispielsweise in einer kunstoffbeschichteten Frotteetasche einer Bandage befindet, beim Beugen des Knies verhindert wird. Bevorzugt sind insbesondere die Flachseiten des Stabilisierungsstabes aufgeraut.

Bevorzugt weist der erste und/oder der zweite Abschnitt des Stabilisierungsstabs, insbesondere der erste Abschnitt des Stabilisierungsstabs ein Griffstück auf. Bevorzugt ist das Griffstück als Öse ausgebildet. Bevorzugt weist die Öse an ihrer dem des Stabilisierungsstab abgewandten Seite eine Verdickung auf. Als Alternative zu einer Öse kann das mindestens eine Griffstück auch Noppen auf dem Stabilisierungsstab aufweisen, die ein Greifen des Stabs beim An- und Ausziehen vereinfachen.

In einer bevorzugten Ausführungsform weist der erste und der zweite Abschnitt des Stabilisierungsstabs jeweils ein Griffstück auf. Bevorzugt sind beide Griffstücke als Öse ausgebildet. In dieser Ausführungsform kann in vorteilhafter Weise das erste Griffstück als Anziehhilfe und das zweite Griffstück als Ausziehhilfe verwendet werden.

Der Stabilisierungsstab wird hierbei mit doppelter Wirkung ausgenutzt, nämlich einerseits zur Stabilisierung des Kniegelenks und andererseits als Anziehhilfe und Ausziehilfe, wozu der Stabilisierungsstab mit mindestens einem Griffstück versehen ist, das leicht erfassbar ist und einen auf ihn ausgeübten Zug direkt auf das Bandagenmaterial überträgt. Es kann am oberen und/oder unteren Ende des Stabilisierungsstabs ausgebildet sein. Beim Zug an dem Griffstück wird dieser auf diese Weise über die gesamte Länge der Bandage in diese eingeleitet. Das Griffstück wird zweckmäßig als Öse ausgebildet, bei dem der Durchgang durch sein Loch etwa rechtwinkelig zum Bandagenmaterial liegt. Bei einer solchen Ausbildung des Griffstücks kann dieses mit einem Finger direkt erfasst werden, der dabei die Öse durchsetzt und den Zug auf diese Weise bequem auf die Bandage überträgt. Das Erfassen der Öse lässt sich weiterhin dadurch erleichtern, dass diese an ihrer dem Stabilisierungsstab abgewandten Seite eine Verdickung aufweist. Auf diese Weise kann die Bandage zum An- und ausziehen leichter mit den Fingern gegriffen werden.

Es zeigte sich überraschender Weise, dass der Stabilisierungsstab trotz eines verjüngten dritten Abschnitts mit mehreren Materialaussparungen dennoch stabil genug ist um auch als Anziehhilfe verwendet werden zu können.

Bevorzugt handelt es sich bei dem erfindungsgemäßen Stabilisierungsstab um einen Stabilisierungsstab für eine Bandage. Bevorzugt handelt es sich bei dem erfindungsgemäßen Stabilisierungsstab um einen Stabilisierungsstab für eine Kniegelenkbandage.

Die vorliegende Erfindung betrifft auch die Verwendung eines erfindungsgemäßen Stabilisierungsstabs in einem orthopädischen Hilfsmittel, insbesondere einer Bandage, bevorzugt einer Kniegelenkbandage umfassend einen Stabilisierungsstab nach einem der vorhergehenden Ansprüche.

Die vorliegende Erfindung betrifft auch ein orthopädisches Hilfsmittel, umfassend einen erfindungsgemäßen Stabilisierungsstab. Bevorzugt ist das orthopädische Hilfsmittel eine Bandage. Besonders bevorzugt ist das orthopädische Hilfsmittel eine Kniebandage beziehungsweise Kniegelenksbandage. Bevorzugt ist der Grundkörper der Kniebandage beziehungsweise Kniegelenksbandage aus einem Textil, insbesondere einem Gestrick oder Gewirk gebildet. Geeignete Kniebandagen beziehungsweise Kniegelenksbandagen und deren Grundkörper sind dem Fachmann bekannt.

Die vorliegende Erfindung betrifft daher auch eine Kniegelenksbandage, umfassend einen erfindungsgemäßen Stabilisierungsstab.

Bevorzugt ist eine Kniegelenksbandage, wobei sich der dritte biegsame Abschnitt des Stabilisierungsstabs im angelegten Zustand der Kniegelenksbandage auf der Höhe des Knies befindet.

Bevorzugt weist die Kniegelenksbandage zwei Stabilisierungsstäbe auf, insbesondere zwei erfindungsgemäße Stabilisierungsstäbe.

Bevorzugt ist eine Kniegelenksbandage, wobei die Kniegelenksbandage zwei Stabilisierungsstäbe, insbesondere zwei erfindungsgemäße Stabilisierungsstäbe, aufweist, wobei sich die Stabilisierungsstäbe über die Länge der Kniegelenksbandage erstrecken. Bevorzugt erstecken sich die Stabilisierungsstäbe seitlich des Knies über die Länge der Kniegelenksbandage.

Bevorzugt ist eine Kniegelenksbandage, wobei sich jeweils der dritte biegsame Abschnitt der Stabilisierungsstäbe im angelegten Zustand der Kniegelenksbandage auf der Höhe des Knies befindet.

Bevorzugt sind die Materialaussparungen nur auf einer Seite des dritten Abschnitts. Bevorzugt sind die Materialaussparungen dabei auf der Seite des dritten Abschnitts, die vom Knie weg zeigt. Dies führt in vorteilhafter Weise dazu, dass bei der Beugung des Knies der dritte Abschnitt so gebogen wird, dass die zwischen den Materialaussparungen liegenden zahnartigen Elemente nach einer bestimmten Beugungsstrecke zusammenstoßen und somit die Bewegung limitieren.

Bevorzugt ist der mindestens eine Stabilisierungsstab in einer an der Bandage angeordneten Tasche eingebettet. Bevorzugt ist ein Teilbereich des mindestens einen Stabilisierungsstabs mit dem Textil der Bandage und/oder der Tasche verschweißt.

Bevorzugt ist eine aus elastischem Material, insbesondere einem Textil bestehende Bandage, insbesondere Kniegelenksbandage, die auf mindestens einer Seite mit einem erfindungsgemäßen sich über die Länge der Bandage erstreckenden Stabilisierungsstab versehen ist, wobei der Stabilisierungsstab mit einem oder zwei Griffstücken versehen und in eine an der Bandage angeordneten Tasche eingebettet ist, die über Randzonen und an ihrem oberhalb der Kniescheibe angeordneten Ende mit dem Material der Bandage fest verbunden ist. Bevorzugt ist der Stabilisierungsstab im Wesentlichen durchgehend mit dem Material der Bandage verschweißt. Bevorzugt ist das Griffstück als Öse ausgebildet, bei dem der Durchgang durch sein Loch etwa rechtwinklig zum Bandagenmaterial liegt. Bevorzugt weist die an ihrer dem Stabilisierungsstab abgewandten Seite eine Verdickung auf.

Bevorzugt weist die Kniegelenksbandage ein der Kniescheibe zugeordnetes Polster auf.

Weitere bevorzugte Ausführungsformen ergeben sich aus den Unteransprüchen, den Beispielen und den Figuren.
- Figur 1: zeigt einen erfindungsgemäßen Stabilisierungsstab;
- Figur 2: zeigt eine erfindungsgemäße Kniegelenksbandage mit zwei Stabilisierungsstäben aus Figur 1;
- Figur 3: zeigt eine alternative Ausführungsform des erfindungsgemäßen Stabilisierungsstabs;
- Figur 4: zeigt den Stabilisierungsstab aus Figur 3 in anderer Perspektive;
- Figur 5: zeigt den mittleren Ausschnitt des Stabilisierungsstabs aus Figur 3;
- Figur 6: zeigt verschiedene Ausführungsformen des Querschnitts des mittleren Bereichs eines erfindungsgemäßen Stabilisierungsstabs.

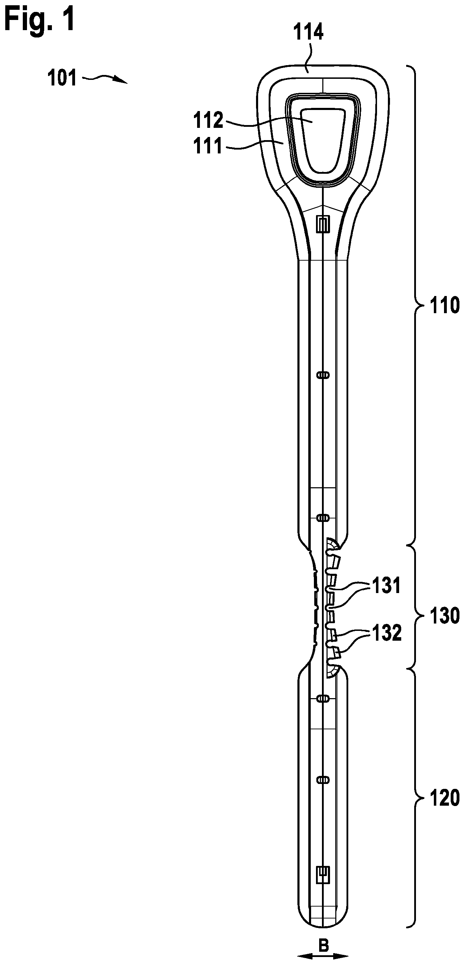

Figur 1 zeigt einen erfindungsgemäßen Stabilisierungsstab 101. Der Stabilisierungsstab 101 ist einstückig ausgebildet und aus einem Kunststoff geformt. Erfindungsgemäß unterteilt sich der Stabilisierungsstab 101 in einen ersten Abschnitt 110, einen zweiten Abschnitt 120 und einen dritten Abschnitt 130, der den ersten Abschnitt 110 und den zweiten Abschnitt 120 miteinander verbindet. Der dritte Abschnitt 130 ist gegenüber dem ersten und dem zweiten Abschnitt 110, 120 in seiner Breite B verjüngt und weist darüber hinaus sieben Materialaussparungen 131 auf. Dabei sind die Materialaussparungen 131 umso tiefer, je weiter sie vom Mittelbereich des dritten biegsamen Abschnitts 130 entfernt sind Dadurch ist der dritte Abschnitt 130 des Stabilisierungsstabs 101 biegsamer als der erste Abschnitt 110 und der zweite Abschnitt 120. Dabei ist insbesondere eine Biegung des dritten Abschnitts 130 in Richtung der Aussparungen 131 vorgesehen, sodass die zwischen den Aussparungen 131 liegenden zahnartigen Materialerhebungen 132 aneinanderstoßen und dadurch die Biegung abfedern und limitieren.

Ein solcher Stabilisierungsstab kann in vorteilhafter Weise durch die Aufteilung in die drei Abschnitte die Beugebewegung im Schwenkbereich der Knieachse ermöglichen, in den anderen Bereichen aber eher stabil und versteifend bleiben. Es wird eine definierte Biegestelle bei gleichzeitigem Festlegen des maximalen Beugewinkels ermöglicht.

Der erste Abschnitt 110 des Stabilisierungsstabs 101 weist ein Griffstück 111 auf, das ein Loch 112 bildet. Das Griffstück 111 ist Bestandteil des ersten Abschnitts 110 und einstückig aus dessen Material gebildet. Der obere Bereich des Griffstücks 111 weist eine Verdickung 114 auf, durch die das Griffstück 111 leichter erfasst und gezogen werden kann.

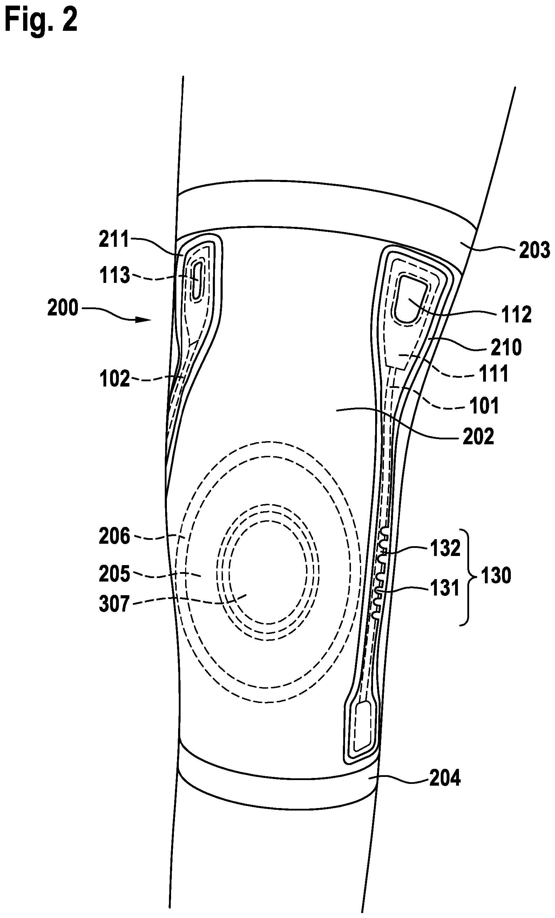

Die in der Figur 2 dargestellte Kniegelenkbandage 200 besteht aus dem aus elastischem Textilmaterial bestehenden Strumpf 202 und sie ist mit den beiden Rändern 203 und 204 an ihren beiden Enden versehen, die zur Rutschsicherung der Bandage 200 beitragen. Außerdem sind diese Ränder 203 und 204 aus einem Material hergestellt, das eine geringere Spannung als der Strumpf 202 aufweist, um das Bein des Trägers an den betreffenden Stellen nur wenig einzuschnüren. Auf der Vorderseite des Kniegelenks ist in dem Strumpf 202 eine Profileinlage mit dem Polster 205 eingearbeitet, die zum Beispiel aus Schaumstoff oder Silikon bestehen kann und eine erhebliche Elastizität besitzt. Das Polster 205 ist auf der Innenseite des Strumpfes 202 durch einen Überzug abgedeckt, der an seinen Rändern 206 mit dem Strumpf 202 verbunden ist, beispielsweise durch Kleben. Das Polster 205 lässt in ihrem mittleren Teil einen Bereich frei, in dem etwa die Kniescheibe 307 hineinpasst. Die Kniescheibe 307 wird somit von dem Polster 205 umfasst. Insoweit handelt es sich um eine in bekannter Weise gestaltete Kniegelenkbandage. Neben dem Polster 205 ist die Bandage 200 mit zwei erfindungsgemäßen Stabilisierungsstäben 101 und 102 versehen, die sich im Wesentlichen über die gesamte Länge der Bandage 200 erstrecken und die dafür sorgen, dass sich die am Bein angelegte Bandage 200 nicht hinsichtlich ihrer Längsrichtung zusammenziehen kann. Jeder der beiden Stabilisierungsstäbe 101 und 102 ist in eine auf die Bandage 200 mittels der Randzone 210 bzw. 211 an das Material der Bandage 200 angeklebte Tasche aufgenommen. Je nach gewünschter Stabilisierungsintensität kann die Bandage 200 auch nur mit einem Stabilisierungsstab versehen sein. Jeder der beiden Stabilisierungsstäbe 101 und 102 weist an seinem oberen Ende ein eine Öse 112 bzw. 113 enthaltendes Griffstück 111 auf, welches das Erfassen der Bandage 200 bei ihrem Anziehen und Hochziehen entlang des Beines mit dem Finger ermöglicht und damit das Anziehen der Bandage 200 erleichtert, da die Bandage 200 bei einem entsprechenden Zug auf die Griffstücke 111 von diesen und dem Stabilisierungsstab 101, 102 insgesamt mitgenommen wird, wodurch es ohne weiteres möglich ist, die Bandage 200 glatt über den Fuß, die Wade und das Knie in ihre endgültige Lage zu ziehen. Die in den Taschen enthaltenen Stabilisierungsstäbe 101 und 102 werden dadurch satt von den betreffenden Taschen umfasst, dass deren Randzone 210 und 211 jeweils als schmaler umlaufener Streifen ausgebildet ist, der direkt mit dem Material der Bandage 200 verbunden ist, zum Beispiel durch verschweißen oder durch verkleben.

Auf die besondere erfindungsgemäß bevorzugte Gestaltung der Stabilisierungsstäbe 101, 102 wird in Figur 1 näher eingegangen. Der dritte Abschnitt 130 der Stabilisierungsstäbe 101, 102 befindet sich dabei auf Höhe der Kniescheibe 307. Somit wird beim Beugen des Knies auch genau dieser dritte Abschnitt 130 gebogen. Dabei sind die Stabilisierungsstäbe 101, 102 so an der Kniegelenkbandage 200 positioniert, dass die Aussparungen 131 nach hinten zeigen. Somit werden beim Beugen des Knies die zwischen den Aussparungen 131 liegenden zahnartigen Materialabschnitte 132 aufeinander gedrückt, wodurch eine Abfederung des Beugungsvorgangs auftritt.

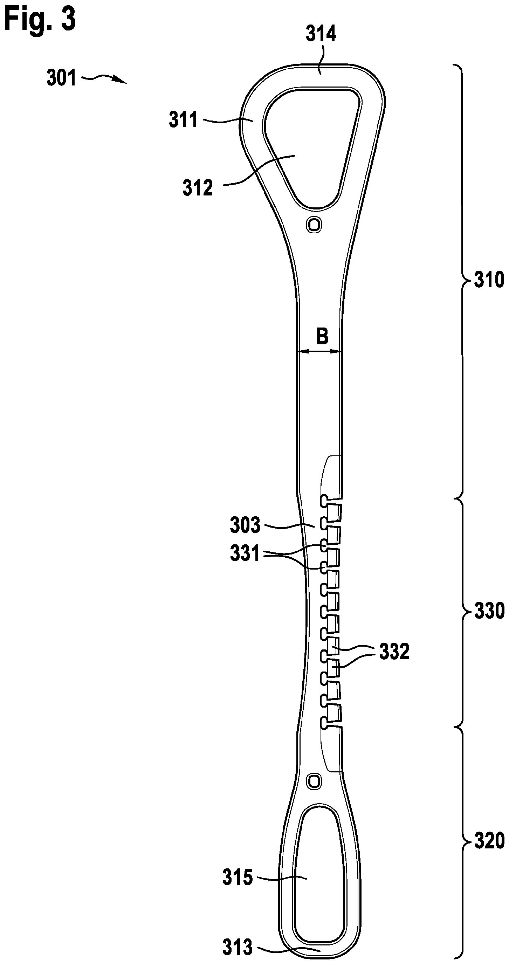

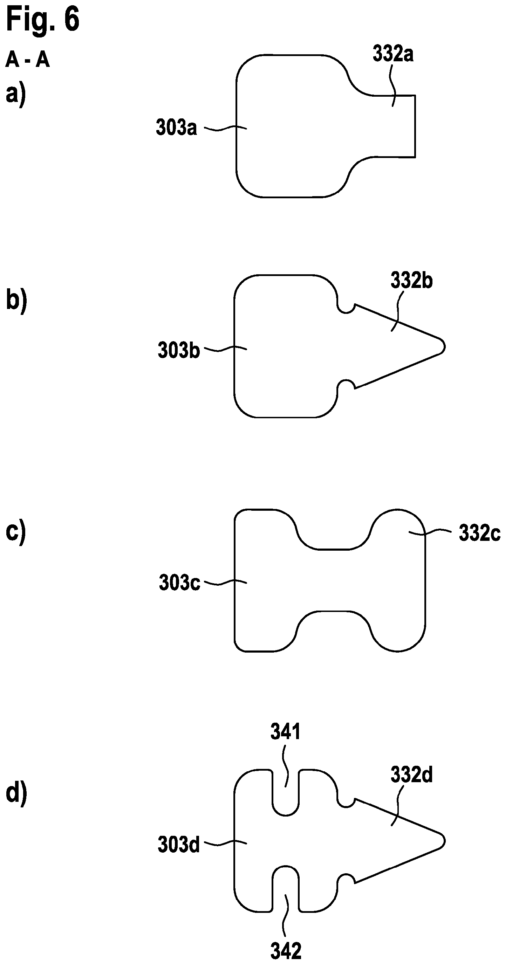

Natürlich kann auch jeder andere erfindungsgemäße Stabilisierungsstab in eine Kniegelenksbandage wie in Figur 2 gezeigt eingearbeitet werden, beispielsweise ein in Figur 3 gezeigter Stabilisierungsstab oder ein Stabilisierungsstab mit einem Querschnitt aus den Figuren 6a bis 6d.

Figur 3 zeigt eine alternative Ausführungsform eines erfindungsgemäßen Stabilisierungsstabs 301. Der Stabilisierungsstab 301 ist ebenfalls einstückig ausgebildet und aus einem Kunststoff geformt. Erfindungsgemäß unterteilt sich der Stabilisierungsstab 301 in einen ersten Abschnitt 310, einen zweiten Abschnitt 320 und einen dritten Abschnitt 330, der den ersten Abschnitt 310 und den zweiten Abschnitt 320 miteinander verbindet. Die Abschnitte werden durch einen Grundkörper 303 gebildet. Der Grundkörper 303 des dritten Abschnitts 330 ist gegenüber dem ersten und dem zweiten Abschnitt 310, 320 in seiner Breite B verjüngt und weist darüber hinaus elf Materialaussparungen 331 auf. Dabei sind die Materialaussparungen 331 umso tiefer, je weiter sie vom Mittelbereich des dritten biegsamen Abschnitts 330 entfernt sind Dadurch ist der dritte Abschnitt 330 des Stabilisierungsstabs 301 biegsamer als der erste Abschnitt 310 und der zweite Abschnitt 320. Dabei ist insbesondere eine Biegung des dritten Abschnitts 330 in Richtung der Aussparungen 331 vorgesehen, sodass die zwischen den Aussparungen 331 liegenden zahnartigen Materialerhebungen 332 aneinanderstoßen und dadurch die Biegung abfedern und limitieren.

Die zahnartigen Materialerhebungen 332 haben eine Hammer-artige Form, so dass die Spalten der Aussparungen 331 am seitlichen Rand des Stabilisierungsstabs 301 enger sind als die runden Enden der Aussparungen 331 im Inneren des Grundkörpers 303 des Stabilisierungsstabs 301. Die gezeigten Aussparungen 331 haben hier eine schlüssellochartige Gesamtform. Darüber hinaus sind die hammerkopfförmigen Enden der zahnartigen Materialerhebungen 332 dicker als der Stielbereich der zahnartigen Materialerhebungen 332. Dies erhöht weiter die Beweglichkeit des dritten Abschnitts 330.

Auch ein solcher Stabilisierungsstab kann in vorteilhafter Weise durch die Aufteilung in die drei Abschnitte die Beugebewegung im Schwenkbereich der Knieachse ermöglichen, in den anderen Bereichen aber eher stabil und versteifend bleiben. Es wird eine definierte Biegestelle bei gleichzeitigem Festlegen des maximalen Beugewinkels ermöglicht.

Der erste Abschnitt 310 des Stabilisierungsstabs 301 weist ein Griffstück 311 auf, das ein Loch 312 bildet. Das Griffstück 311 ist Bestandteil des ersten Abschnitts 310 und einstückig aus dessen Material gebildet. Der obere Bereich des Griffstücks 311 weist eine Verdickung 314 auf, durch die das Griffstück 311 leichter erfasst und gezogen werden kann. Der zweie Abschnitt 320 des Stabilisierungsstabs 301 weist ebenfalls ein Griffstück 313 auf, das ebenfalls ein Loch 315 bildet. Das Griffstück 313 ist Bestandteil des zweiten Abschnitts 320 und einstückig aus dessen Material gebildet.

Die Griffstücke 311 und 313 eignen sich als Anziehhilfe beziehungsweise Ausziehhilfe für eine Kniegelenksbandage, die mindestens einen Stabilisierungsstab 301 aufweist.

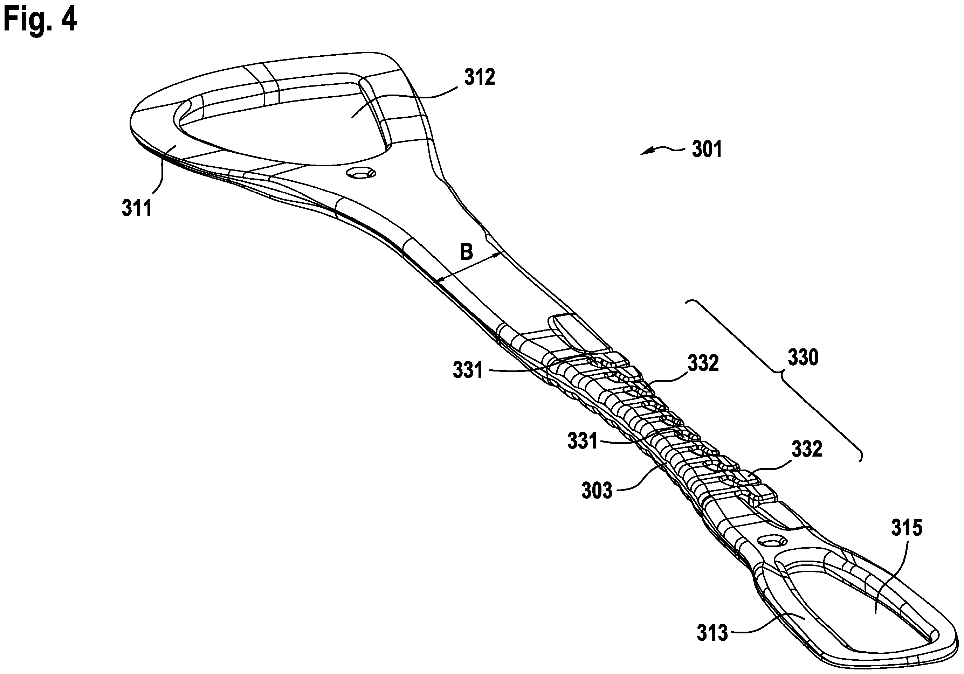

Figur 4 zeigt die Ausführungsform des Stabilisierungsstabs 301 aus Figur 3 in anderer Perspektive. Zu sehen ist wieder der biegsame Mittelabschnitt 330 mit den Aussparungen 331 und den zahnartigen Materialerhebungen 332 sowie die Griffstücke 311 und 313 mit den Löchern 312 und 315. Deutlich zu erkennen ist, dass die Breite B des Grundkörpers 303 des mittleren Abschnitts 330 geringer ist als die Breite B der anderen Abschnitte des Stabilisierungsstabs 301. Der Abschnitt 330 ist somit verjüngt. Zu erkennen ist ebenfalls, dass der Stabilisierungsstabgrundkörper 303 im Bereich 330 wellenförmig ausgestaltet ist, wobei der Grundkörper im Bereich der Aussparungen 331 Erhöhungen aufweist und im Bereich der Materialerhebung 332 Täler aufweist. Dadurch kann die Biegsamkeit des Bereichs 330 beeinflusst werden. Ebenfalls ist zu erkennen, dass sich der Grundkörper des Stabilisierungsstabs 301 im Bereich 330 zu den Materialerhebungen 332 hin verjüngt, der Grundkörper also keilförmig ausgebildet ist. Diese Formausgestaltung fördert das leichtere Einbeugen des Stabes, da die Stauchung des Materials im Bereich der Biegestelle am Größten ist. Somit ist in dieser Ausführungsform dieser Bereich in vorteilhafterweise nicht nur geschlitzt, sondern auch in der Materialdicke verringert.

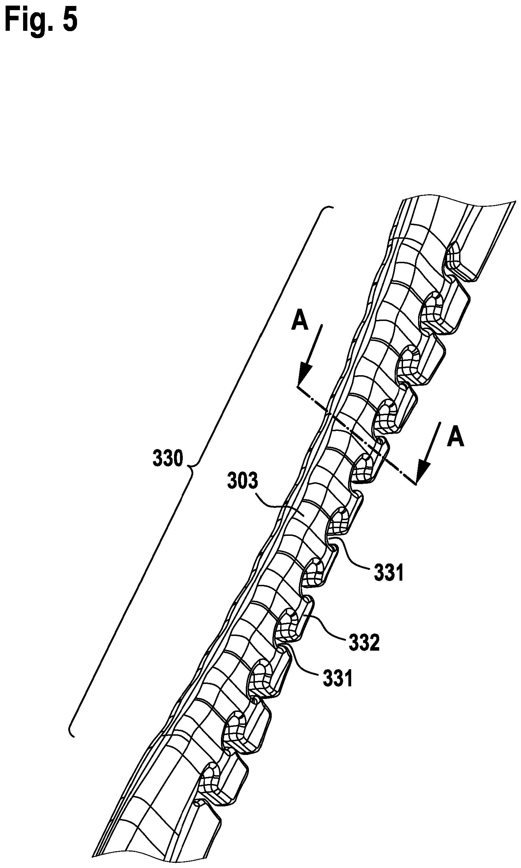

Figur 5 zeigt einen Ausschnitt des Stabilisierungsstabs aus Figur 3 im Bereich des dritten biegsamen Abschnitts 330, der wieder einen Grundkörper 303 und daran anliegende Materialerhebungen 332 aufweist durch die Aussparungen 331 gebildet werden. Die für die Figuren 3 und 4 beschriebenen Formgebungen des Abschnitts 330 und insbesondere des Grundkörpers 303, der Materialerhebung 332 und der Aussparung 331 sind auch hier deutlich zu erkennen.

Gezeigt ist weiterhin eine Querschnittslinie AA. Der Querschnitt AA kann nicht nur die sich aus den Figuren 3 bis 5 ergebende Form haben, sondern auch verschiedene andere Formen.

Solche anderen Formen des Querschnitts A-A durch den Grundkörper und eine der die Aussparungen bildenden Materialerhöhungen sind beispielhaft in Figur 6 gezeigt. In Figur 6a ist ein rechteckiger Grundkörper 303a mit abgerundeten Kanten gezeigt und die Materialerhebung 332a weist ebenfalls eine einfache rechteckige Grundform auf. In Figur 6b weist der Grundkörper 303b wieder eine rechteckige Grundform mit abgerundeten Kanten auf, wobei die zahnartige Materialerhebung 332b pfeilspitzenförmig ausgebildet ist. In Figur 6c ist der Grundkörper 303c mit einer geringeren Materialdicke ausgeformt und die zahnartige Materialerhebung 332c ist pilzkopfartig ausgebildet. In Figur 6d weist der Grundkörper 303d zwei Nuten 341 und 342 auf. Diese Nuten oder Auskerbungen dienen vorteilhafterweise der Minimierung von Spannung im verwendeten Material. Sie helfen darüber hinaus dabei, den Stab nicht nur in der Beugerichtung des Kniegelenks leicht einbiegen zu können, sondern auch um 90 versetzt den Stab beim Ausziehen der Bandage über die flachbandartige Seite des Stabes um 180 biegen zu können. Denn das Ablegen einer Kniebandage wird vom Patienten üblicherweise, insbesondere wenn kein weiterer Griff (wie er in Figur 3 als 313 gezeigt ist) vorhanden ist derart bewerkstelligt, dass der Patient den oberen Rand einer Bandage greift und, gleich dem Ausziehen eines Kniestrumpfs, in Richtung der Ferse zieht. Dabei wird das Material der Bandage um 180° gebogen, um die Bandage dann in der Biegerichtung nach unten zu ziehen. Die durch die Nuten 341 und 342 verbesserte Biegsamkeit des Stabes führt zum erleichterten Ausziehen der Bandage. Die Nuten ermöglichen also das ein Stabilisierungsstab nicht nur in der Beugerichtung durch die Aussparungen im mittleren Bereich gebogen werden kann, sondern auch in die 90 ° versetzte seitliche Richtung. Die zahnartige Materialerhebung 332d ist wieder pfeilspitzenförmig ausgestaltet.

## Patentansprüche

1. Stabilisierungsstab (101) für ein orthopädisches Hilfsmittel (200), wobei der Stabilisierungsstab (101) in Längsrichtung einen ersten Abschnitt (110) und einen zweiten Abschnitt (120) aufweist, wobei der erste Abschnitt (110) und der zweite Abschnitt (120) über einen dritten, biegsamen Abschnitt (130) miteinander verbunden sind, wobei zumindest der dritte, biegsame Abschnitt (130) mehrere Materialaussparungen (131) aufweist, wobei die Materialaussparungen (131) umso tiefer sind, je weiter sie vom Mittelbereich des dritten biegsamen Abschnitts (130) entfernt sind **dadurch gekennzeichnet, dass** die Materialaussparungen durch zwischen den Materialaussparungen liegenden zahnartigen Materialerhebungen gebildet werden.

2. Stabilisierungsstab (101) nach Anspruch 1, wobei der Stabilisierungsstab (101) einstückig ist und aus einem Material, insbesondere einem Kunststoff, besteht.

3. Stabilisierungsstab (101) nach einem der Ansprüche 1 bis 2, wobei in den Stabilisierungsstab (101) eine Sehne aus einem härteren Material als das Material des Stabilisierungsstabs (101), insbesondere eine Sehne aus Metall, eingebettet ist.

4. Stabilisierungsstab (101) nach einem der vorhergehenden Ansprüche, wobei der erste Abschnitt (110) und der zweite Abschnitt (120) weniger biegsam sind als der dritte Abschnitt (130).

5. Stabilisierungsstab (101) nach einem der vorhergehenden Ansprüche, wobei es sich bei dem orthopädischen Hilfsmittel um eine Bandage handelt, insbesondere um eine Kniegelenkbandage (200).

6. Stabilisierungsstab nach einem der vorhergehenden Ansprüche, wobei der Stabilisierungsstab (101) eine Länge, eine Breite und ein Höhe hat, wobei die Breite des dritten biegsamen Abschnitts (130) kleiner ist als die Breite des ersten Abschnitts und des zweiten Abschnitts.

7. Stabilisierungsstab nach einem der vorhergehenden Ansprüche 1-5, wobei der Stabilisierungsstab (101) eine Länge, eine Breite und ein Höhe hat, wobei die Breite des ersten Abschnitts (110) und die Breite des zweiten Abschnitts (120) größer ist als die Höhe des Stabilisierungsstabes (101) und wobei die Breite des dritten, biegsamen Abschnitts (130) kleiner ist als die Breite des ersten Abschnitts (110) und die Breite des zweiten Abschnitts (120).

8. Orthopädisches Hilfsmittel, umfassend einen Stabilisierungsstab (101) nach einem der vorhergehenden Ansprüche.

9. Orthopädisches Hilfsmittel nach Anspruch 8, wobei das orthopädische Hilfsmittel eine Kniegelenksbandage (200) ist.

10. Orthopädisches Hilfsmittel (200) nach Anspruch 9, wobei die Kniegelenksbandage (200) zwei Stabilisierungsstäbe (101, 102) nach einem der Ansprüche 1 bis 7 aufweist, wobei sich die Stabilisierungsstäbe (101, 102) über die Länge der Kniegelenksbandage (200) erstrecken.

11. Orthopädisches Hilfsmittel (200) nach Anspruch 9 oder 10, wobei sich jeweils der dritte biegsame Abschnitt (130) der Stabilisierungsstäbe (101. 102) im angelegten Zustand der Kniegelenksbandage (200) zumindest auf der Höhe des Knies (307) befindet.

## Claims

1. Stabilising bar (101) for an orthopaedic aid (200), the stabilising bar (101) having a first portion (110) and a second portion (120) in the longitudinal direction, the first portion (110) and the second portion (120) being interconnected by a third flexible portion (130), wherein at least the third flexible portion (130) has a plurality of material recesses (131), wherein the material recesses (131) are deeper the further away they are from the central region of the third flexible portion (130), **characterised in that** the material recesses are formed by tooth-like material protrusions located between the material recesses.

2. Stabilising bar (101) according to claim 1, wherein the stabilising bar (101) is made of one piece and consists of a material, in particular a plastic.

3. Stabilising bar (101) according to any one of claims 1 to 2, wherein a chord made of a harder material than the material of the stabilising bar (101), in particular a chord made of metal, is embedded in the stabilising bar (101).

4. Stabilising bar (101) according to any one of the preceding claims, wherein the first portion (110) and the second portion (120) are less flexible than the third portion (130).

5. Stabilising bar (101) according to any one of the preceding claims, wherein the orthopaedic aid is a bandage, in particular a knee joint bandage (200).

6. Stabilising bar according to any one of the preceding claims, wherein the stabilising bar (101) has a length, a width and a height, wherein the width of the third flexible portion (130) is smaller than the width of the first portion and the second portion.

7. Stabilising bar according to any one of the preceding claims 1-5, wherein the stabilising bar (101) has a length, a width and a height, wherein the width of the first portion (110) and the width of the second portion (120) is greater than the height of the stabilising bar (101) and wherein the width of the third flexible portion (130) is smaller than the width of the first portion (110) and the width of the second portion (120).

8. Orthopaedic aid comprising a stabilising bar (101) according to any one of the preceding claims.

9. Orthopaedic aid according to claim 8, wherein the orthopaedic aid is a knee joint bandage (200).

10. Orthopaedic aid (200) according to claim 9, wherein the knee joint bandage (200) comprises two stabilising bars (101, 102) according to any one of claims 1 to 7, wherein the stabilising bars (101, 102) extend along the length of the knee joint bandage (200).

11. Orthopaedic aid (200) according to claim 9 or 10, wherein the third flexible portion (130) of the stabilising bars (101, 102) is located at least at the level of the knee (307) in the applied state of the knee joint bandage (200).

## Revendications

1. Barre de stabilisation (101) pour un appareil orthopédique (200), la barre de stabilisation (101) présentant dans la direction longitudinale une première section (110) et une deuxième section (120), la première section (110) et la deuxième section (120) étant reliées l'une à l'autre par une troisième section flexible (130), au moins la troisième section flexible (130) présente plusieurs évidements de matériau (131), les évidements de matériau (131) étant d'autant plus profonds qu'ils sont éloignés de la zone centrale de la troisième section flexible (130), **caractérisé en ce que** les évidements de matériau sont formés par des saillies de matériau en forme de dents situées entre les évidements de matériau.

2. Barre de stabilisation (101) selon la revendication 1, dans laquelle la barre de stabilisation (101) est d'une seule pièce et est constituée d'un matériau, en particulier d'un plastique.

3. Barre de stabilisation (101) selon l'une des revendications 1 à 2, dans laquelle une corde en un matériau plus dur que le matériau de la barre de stabilisation (101), en particulier une corde en métal, est incorporée dans la barre de stabilisation (101).

4. Barre de stabilisation (101) selon l'une quelconque des revendications précédentes, dans laquelle la première section (110) et la deuxième section (120) sont moins flexibles que la troisième section (130).

5. Barre de stabilisation (101) selon l'une quelconque des revendications précédentes, dans laquelle l'appareillage orthopédique est un bandage, en particulier un bandage d'articulation du genou (200).

6. Barre de stabilisation selon l'une quelconque des revendications précédentes, dans laquelle la barre de stabilisation (101) a une longueur, une largeur et une hauteur, la largeur de la troisième partie flexible (130) étant inférieure à la largeur de la première partie et de la deuxième partie.

7. Barre de stabilisation selon l'une quelconque des revendications précédentes 1-5, dans laquelle la barre de stabilisation (101) a une longueur, une largeur et une hauteur, dans laquelle la largeur de la première section (110) et la largeur de la deuxième section (120) sont supérieures à la hauteur de la barre de stabilisation (101) et dans laquelle la largeur de la troisième section flexible (130) est inférieure à la largeur de la première section (110) et à la largeur de la deuxième section (120).

8. Appareillage orthopédique comprenant une barre de stabilisation (101) selon l'une des revendications précédentes.

9. Appareillage orthopédique selon la revendication 8, dans laquelle l'appareillage orthopédique est un bandage de l'articulation du genou (200).

10. Appareillage orthopédique (200) selon la revendication 9, dans laquelle le bandage de l'articulation du genou (200) comporte deux barres de stabilisation (101, 102) selon l'une des revendications 1 à 7, les barres de stabilisation (101, 102) s'étendant sur la longueur du bandage de l'articulation du genou (200).

11. Appareillage orthopédique (200) selon la revendication 9 ou 10, dans lequel la troisième section flexible (130) de chacune des barres de stabilisation (101, 102) se trouve au moins au niveau du genou (307) lorsque le bandage de l'articulation du genou (200) est en place.
